# FASCICULE DE BREVET EUROPEEN

(11) **EP 0 680 944 B1**
(45) Date de publication et mention de la délivrance du brevet: **25.02.1998**
(21) Numéro de dépôt: 95400885.0
(22) Date de dépôt: 20.04.1995
(51) Int. Cl.: C07C 43/04, C07C 41/42, C07C 41/06

(54) **Procédé de purification d'éther comprenant deux étapes de distillation**
Zwei Destillationschritte enthaltendes Etherreinigungsverfahren
Ether purification process comprising two distillation steps

(30) Priorité: 05.05.1994 FR 9405657
(43) Date de publication de la demande: 08.11.1995
(73) Titulaire: INSTITUT FRANCAIS DU PETROLE, 92502 Rueil-Malmaison (FR)
(72) Inventeur: Streicher, Christian, F-92500 Rueil Malmaison (FR)

(56) Documents cités:
- EP-A- 0 497 680
- EP-A- 0 507 076
- EP-A- 0 542 596

## Description

### Domaine de l'invention

L'invention concerne la fabrication des éthers à partir de monoalcools aliphatiques à au moins deux atomes de carbone et d'isooléfines à au moins 4 atomes de carbone et plus particulièrement la fabrication de l'éthyl tertiobutyl éther (en abrégé ETBE).

### État de l'art

On sait que les éthers comme l'éthyl tertiobutyl éther, ou comme le méthyl tertiobutyl éther (en abrégé MTBE), peuvent être utilisés comme additifs à haut indice d'octane pour les essences sans plomb ou à teneur en plomb réduite. On peut prévoir d'ajouter l'ETBE aux essences à des concentrations allant par exemple jusqu'à environ 15 % en volume.

Un procédé de fabrication du MTBE consiste à réaliser une réaction d'addition de méthanol sur de l'isobutène, par exemple contenu dans une coupe C₄ de vapocraquage ou de craquage catalytique. Après réaction, le méthanol résiduel est en général séparé par distillation azéotropique avec les hydrocarbures en C₄, ce qui permet d'obtenir aisément le MTBE avec un degré de pureté convenable pour être additionné aux essences.

La fabrication d'éthers à partir de monoalcools aliphatiques à au moins 2 atomes de carbone peut être réalisée par un procédé analogue, dans lequel de tels alcools remplacent le méthanol. On peut ainsi produire, par exemple, de l'ETBE à partir d'éthanol et d'isobutène, mais également d'autres éthers comme par exemple l'isopropyl tertiobutyl éther (en abrégé IPTBE),à partir d'isopropanol et d'isobutène, ou l'éthyl tertioamyl éther (en abrégé ETAE), à partir d'éthanol et d'isoamylènes. Un tel procédé pour la synthèse d'ETBE est décrit par exemple dans "l'ETBE, un avenir pour l'éthanol" de A. FORESTIERE, B. TORCK et G. PLUCHE, communication faite à la Conférence sur la Biomasse pour l'Energie et l'Industrie, Lisbonne, 9-13 Octobre 1989 et dans "MTBE/ETBE, an Incentive Flexibility for Refiners" par A. FORESTIERE et coll., communication faite à la "Conférence on Oxygenated Fuels in Europe", Londres, 22-23 Mai 1990.

Cependant, par un tel procédé, contrairement au cas du MTBE, après élimination des hydrocarbures en C₄, par distillation azéotropique (débutaniseur), une part importante de l'éthanol résiduel se retrouve en mélange avec l'ETBE produit en fond du débutaniseur. L'existence d'un azéotrope éthanol-ETBE à 21 % en poids d'éthanol à la pression atmosphérique, bouillant à 66,6°C, rend difficile la séparation de l'ETBE avec un degré de pureté suffisant pour satisfaire les spécifications concernant la teneur en éthanol des essences. Ainsi la teneur en éthanol de l'ETBE doit être généralement comprise entre 0,1 et 10% en poids. Avantageusement, l'ETBE devra être purifié à moins de 1 % en poids d'éthanol pour être additionné aux essences.

Ainsi, pour que l'ETBE puisse concurrencer le MTBE comme additif améliorant l'indice d'octane des essences sans plomb, il était particulièrement souhaité de trouver un procédé de séparation économiquement attractif.

Divers procédés de purification de l'ETBE obtenu en fond du débutaniseur ont été proposés.

Ainsi, le brevet FR-B-2683523 propose-t-il un procédé dans lequel le mélange alcools/ETBE, obtenu en fond du débutaniseur, est lavé à l'eau, l'extrait eau/alcools ainsi obtenu est ensuite concentré dans une première colonne de distillation puis déshydraté dans deux autres colonnes de distillation hétéroazéotropique utilisant l'ETBE comme agent azéotropant. Ce procédé est cependant relativement complexe et coûteux puisqu'il nécessite l'emploi de 4 colonnes (1 colonne de lavage, 1 colonne de concentration, 2 colonnes de distillation hétéroazéotropique). Il a de plus l'inconvénient de produire un ETBE saturé en eau, ce qui n'est pas avantageux du point de vue de son utilisation comme additif aux essences.

Un procédé plus simple de séparation de mélanges éthanol/ETBE est proposé dans le brevet FR-B-2672048.

Dans ce procédé, on utilise le changement de la composition du mélange azéotropique éthanol/ETBE avec la pression. Le mélange éthanol/ETBE est donc séparé par deux colonnes de distillation opérées sous deux pressions différentes. On obtient ainsi l'ETBE pur en fond de la première colonne opérée sous haute pression et l'éthanol pur en fond de la seconde colonne opérée sous basse pression. Les mélanges azéotropiques obtenus en tête de chaque colonne étant recyclés vers l'autre colonne.

Le brevet FR-B-2673624 décrit lui un procédé de séparation de mélanges éthanol/ETBE par distillation hétéroazéotropique utilisant l'eau comme agent azéotropant. Dans ce procédé on utilise également deux colonnes de distillation pouvant être opérées sous deux pressions différentes et produisant respectivement l'éthanol et l'ETBE pur en fond de chacune d'elles, de manière analogue au procédé précédent.

Dans les deux procédés précédemment décrits on est cependant amené à mettre en oeuvre deux colonnes de distillation, ce qui les rend là encore relativement coûteux en investissements ainsi qu'en consommation énergétique. De plus, comme il est mentionné dans le brevet FR-B-2672048, les mélanges éthanol/ETBE obtenus en fond du débutaniseur dans les procédés de synthèse d'ETBE contiennent d'autres impuretés comme par exemple l'alcool butylique tertiaire (en abrégé ABT), le diéthyléther (en abrégé DEE), les hydrocarbures ayant au moins 5 atomes de carbone (en abrégé C₅+) ou l'éthyl 2-butyl éther (en abrégé E2BE). Or certaines de ces impuretés (DEE, C₅+) sortent en tête des colonnes de distillation dans les procédés des brevets FR-B-2672048 et FR-B-2673624. Les produits de tête étant entièrement recyclés dans ces procédés, ces impuretés vont s'accumuler progressivement jusqu'à perturber le bon fonctionnement du procédé et dégrader la qualité de la séparation effectuée, ce qui nécessite l'emploi d'une purge en tête de l'une ou l'autre colonne pour pallier cet inconvénient.

### Objet de l'invention

La présente invention a donc pour objet un procédé de purification d'éthers, obtenus à partir de monoalcools aliphatiques ayant au moins deux atomes de carbone et d'isooléfines ayant au moins 4 atomes de carbone, combinant l'étape de distillation azéotropique habituellement présente après la section réactionnelle dans les procédés de synthèse d'éthers avec une autre étape de distillation de manière à obtenir l'éther en fond de ladite colonne de distillation azéotropique essentiellement exempt d' alcool. L'éther ainsi obtenu peut contenir moins de 1 % poids voire moins de 0,1 % poids d'alcools résiduels.

La présente invention a plus particulièrement pour objet un procédé de purification de l'ETBE combinant l'étape de distillation azéotropique (débutaniseur) habituellement présente après la section réactionnelle dans les procédés de synthèse d'ETBE avec une autre étape de distillation de manière à obtenir l'ETBE en fond du débutaniseur essentiellement exempt des alcools (éthanol, ABT) présents dans l'effluent de la section réactionnelle. L'ETBE ainsi obtenu peut contenir moins de 1 % poids voire moins de 0,1 % poids d'alcools résiduels.

Le procédé de la présente invention peut s'appliquer aussi bien lorsque l'étape de distillation azéotropique est constituée d'une colonne de distillation conventionnelle que lorsqu'il s'agit d'une étape de distillation catalytique ou réactive, telle celles décrites dans "La distillation réactive: principe, applications et perspectives", par P. MIKITENKO, publié dans *Pétrole et techniques* , n° 329, décembre 1986, p. 34-38.

La présente invention a également pour objet un procédé de synthèse d'éther obtenu à partir d'un monoalcool aliphatique ayant au moins deux atomes de carbone et d'une isooléfine ayant au moins 4 atomes de carbone, incluant le procédé de purification de l'éther de la présente invention, dans lequel l'alcool est recyclé vers la section réactionnelle ou éventuellement vers la distillation catalytique ou réactive lorsqu'un tel procédé est utilisé.

Le procédé de la présente invention s'adresse plus particulièrement aux mélanges d'ETBE d'éthanol et d'hydrocarbures en C₄ constituant les effluents des sections réactionnelles des procédés de synthèse d'ETBE.

Ces mélanges contiennent généralement de 0,1 à 20% poids, préférentiellement de 0,5 à 5 % poids d'éthanol et de 5 à 80 % poids, préférentiellement de 10 à 50 % poids d'ETBE, le reste étant principalement constitué d'hydrocarbures à 4 atomes de carbone (C₄). Ils contiennent également en général, à l'état d'impuretés, jusqu'à 1 % poids d'eau, jusqu'à 1 % poids d'ABT, jusqu'à 1 % poids de DEE, jusqu'à 1 % poids d'éthyl 2-butyl éther (en abrégé E2BE), jusqu'à 5 % poids, préférentiellement moins de 1 % poids d'hydrocarbures ayant au moins 5 atomes de carbone (C₅+) et jusqu'à 5 % poids d'hydrocarbures à 3 atomes de carbone (C₃).

### Description de l'invention

Le mélange contenant l'ETBE, l'éthanol, les hydrocarbures (C₄) et lesdites impuretés est introduit dans le débutaniseur. Le débutaniseur est opéré sous une pression généralement supérieure à la pression atmosphérique, de préférence comprise entre 5 et 15 bar. On recueille en tête dudit débutaniseur un distillat contenant l'essentiel des C₃, des C₄ et de l'eau du mélange à séparer, une fraction de l'éthanol et des C₅+ du mélange à séparer et essentiellement exempt des autres composés (ETBE, E2BE, ABT, DEE). On recueille en fond dudit débutaniseur l'essentiel de l'ETBE et des autres éthers (E2BE, DEE) avec une fraction des C₅+ et de faibles quantités (moins de 1 % poids, préférentiellement moins de 0,1 % poids) d'alcools (éthanol, ABT).

On soutire latéralement dudit débutaniseur au moins une phase vapeur, liquide ou mixte, que l'on envoie dans une seconde zone de distillation.

Selon une caractéristique avantageuse du procédé, on effectue un soutirage latéral d'au moins une phase d'au moins un plateau du débutaniseur dans lequel la concentration en éthanol est sensiblement maximale.

Selon une autre caractéristique avantageuse du procédé, la seconde zone de distillation est opérée sous une pression inférieure à celle du débutaniseur.

On recueille ainsi en fond de cette seconde zone de distillation le reste de l'éthanol contenu dans le mélange à séparer introduit dans le débutaniseur, en mélange avec l'essentiel de l'ABT, les autres produits (éthers, hydrocarbures) n'étant présents qu'à l'état d'impuretés (moins de 1 % poids en général). Ce mélange d'alcools peut alors avantageusement être recyclé vers la section réactionnelle d'un procédé de synthèse d'ETBE.

En tête de la seconde zone de distillation, on obtient un mélange d'éthanol et d'ETBE avec des proportions variables des autres produits (hydrocarbures, ABT, DEE, E2BE) que l'on recycle dans le débutaniseur. Ce mélange peut avantageusement être recyclé à un plateau du débutaniseur inférieur ou égal à celui du soutirage latéral et de préférence à un plateau où la composition est sensiblement la même que celle de ce mélange.

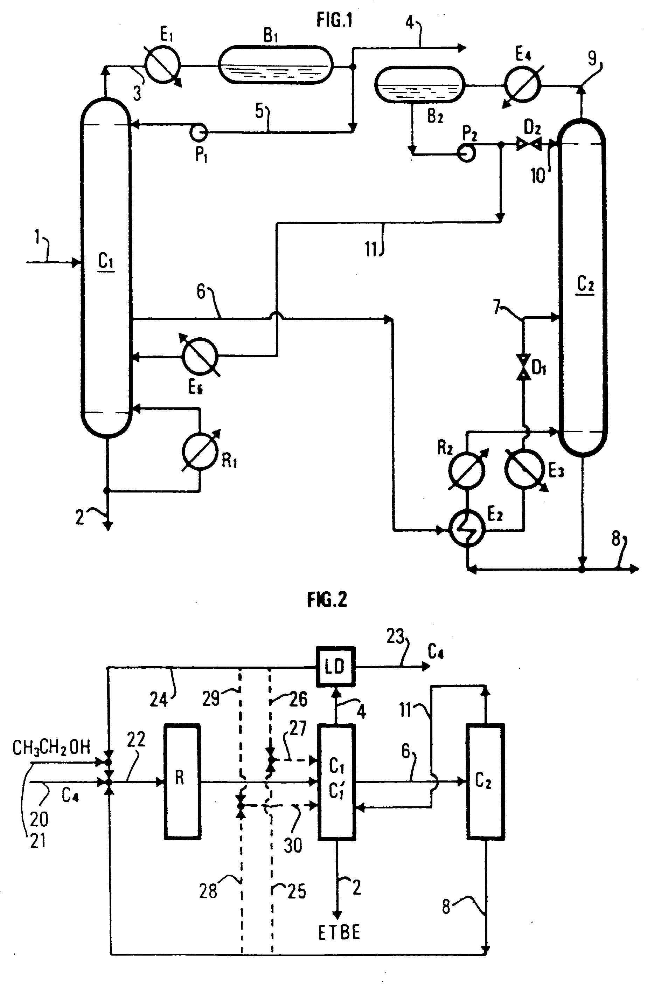

On décrit un mode avantageux de réalisation de l'invention en relation avec la figure 1.

Le mélange à séparer est envoyé par la ligne 1 dans la colonne de distillation C1 (débutaniseur). Cette colonne opère généralement sous une pression p1 supérieure à 1 bar, préférentiellement comprise entre 5 et 15 bar. Elle est chauffée par le rebouilleur R1. La température de fond est en général comprise entre 70 et 200°C. La température de tête est en général comprise entre 30 et 100°C. Le mélange à séparer est introduit dans le débutaniseur C1 préférentiellement au point de bulle à la pression considérée au plateau ayant la composition la plus voisine possible de celle dudit mélange, par exemple dans la partie médiane de la colonne dans le cas de mélanges contenant de 10 à 50 % en poids d'ETBE.

Le produit sortant en fond du débutaniseur par la ligne 2 est constitué de l'ETBE purifié. La quasi-totalité des éthers (ETBE, mais également E2BE et DEE) présents dans le mélange à séparer sont ainsi récupérés, essentiellement exempts de C₃, de C₄ et d'eau, à une teneur en C₅+ fonction de celle du mélange à séparer, usuellement comprise entre 0,1 et 1 % en poids et à des teneurs résiduelles en alcools (éthanol et ABT) fonctions des conditions opératoires du procédé, généralement inférieures à 1 % en poids et pouvant même être inférieures à 0,1 % en poids.

En tête du débutaniseur on récupère par la ligne 3 un distillat en phase vapeur constitué d'hydrocarbures C₃, C₄ et C₅+, contenant une proportion variable d'éthanol, généralement comprise entre 1 et 5 % poids, de l'eau à une teneur généralement inférieure à 1 % poids et essentiellement exempt d'ABT et d'éthers (ETBE, E2BE, DEE).

Cette phase vapeur est entièrement condensée et éventuellement sous-refroidie dans l'échangeur E1 puis admise dans le ballon décanteur B1. Du ballon B1, on recueille par la ligne 4 un distillat liquide de même composition et contenant la quasi-totalité de l'eau et des hydrocarbures C₃ et C₄, ainsi qu'une partie des hydrocarbures C₅+ et de l'éthanol du mélange à séparer. Le reste du liquide recueilli au niveau du ballon B1 est envoyé par la ligne 5, grâce à la pompe P1 en reflux en tête du débutaniseur C1.

Une fraction préférentiellement vapeur est prélevée par la ligne 6 sur un soutirage latéral du débutaniseur C1. Ce soutirage latéral est préférentiellement placé au niveau d'un plateau où la concentration en éthanol dans la phase vapeur est sensiblement maximale, en général à un plateau inférieur à celui de l'alimentation du débutaniseur C1 par le mélange à séparer. Le débit massique de la fraction vapeur ainsi prélevée est un paramètre opératoire important du procédé. Il doit être ajusté en fonction des autres paramètres opératoires et de la teneur en alcools (éthanol, ABT) résiduelle requise dans l'ETBE recueilli en fond du débutaniseur C1.

Cette fraction vapeur est ensuite condensée au moins partiellement dans l'échangeur E2, éventuellement sous-refroidie dans l'échangeur E3, puis détendue à la pression p2 dans la vanne de détente D1 avant d'être admise par la ligne 7 en alimentation de la colonne de distillation C2, opérée sous la pression p2.

Cette pression p2 est inférieure, en général d'au moins 1 bar, préférentiellement de 2 à 10 bar à la pression p1 sous laquelle est opéré le débutaniseur. p2 est alors en général comprise entre 0,5 et 10 bar.

La colonne C2 peut être réchauffée au moins partiellement par l'échangeur E2 (ce qui permet de récupérer au moins une partie de la chaleur de condensation de la vapeur soutirée par la ligne 6), puis par le rebouilleur R2. La température de fond de la colonne C2 est en général comprise entre 70 et 170°C, préférentiellement entre 80 et 120°C. La température de tête de la colonne C2 est en général comprise entre 10 et 130°C, préférentiellement entre 35 et 70°C.

Le produit sortant en fond de la colonne C2 par la ligne 8 est un mélange d'alcools (éthanol, ABT) en proportions variables.

Selon les conditions opératoires de mise en oeuvre du procédé, ce mélange d'alcools peut avoir une teneur en ETBE résiduel inférieure à 1 % en poids et même inférieure à 0,1 % en poids. Il est essentiellement exempt de tous les autres produits (C₃, C₄, C₅+, eau, DEE et E2BE).

En tête de la colonne C2 on récupère par la ligne 9 un distillat en phase vapeur contenant des proportions variables d'éthanol, d'ETBE, d'hydrocarbures C₄ et C₅+, d'ABT et de DEE. Ce distillat est entièrement condensé et éventuellement sous-refroidi dans l'échangeur E4 puis admis au ballon séparateur B2. Du ballon B2, on récupère ce distillat condensé que l'on porte grâce à la pompe P2 à une pression suffisante pour qu'il puisse être retourné au débutaniseur C1. Une fraction dudit distillat est alors renvoyée après détente dans la vanne D2, par la ligne 10, en reflux en tête de la colonne C2. La fraction résiduelle dudit distillat est alors recyclée par la ligne 11 vers le débutaniseur C1 où elle est admise après réchauffage éventuel et éventuellement vaporisation au moins partielle dans l'échangeur E5. La fraction ainsi recyclée au débutaniseur C1 est préférentiellement admise au point de bulle à la pression considérée au plateau dont le liquide a la composition la plus voisine de ladite fraction recyclée, généralement à un plateau inférieur ou égal à celui auquel on effectue le soutirage de la fraction vapeur. Dans les cas où ladite fraction recyclée est au moins partiellement vaporisée il peut être avantageux, après séparation dans un ballon séparateur de la fraction vaporisée et de la fraction liquide de ladite fraction recyclée, d'admettre ladite fraction vapeur dans le débutaniseur C1 à un plateau supérieur à celui auquel est admise ladite fraction liquide et éventuellement supérieur à celui auquel est effectué le soutirage latéral.

### Avantages du procédé

Le procédé de la présente invention a pour principal avantage sa très grande simplicité puisqu'il permet d'obtenir l'ETBE purifié avec une seule colonne de distillation en plus du débutaniseur, qui, lui, est présent dans tous les procédés précédemment décrits de synthèse d'ETBE, alors que tous les autres procédés précédemment décrits de purification d'ETBE nécessitent l'emploi d'au moins deux colonnes en plus dudit débutaniseur. Le procédé de la présente invention s'avère donc particulièrement économe en investissements.

Il présente en outre l'avantage de ne pas permettre l'accumulation des impuretés du mélange éthanol/ETBE/C₄ à séparer (ABT, C₃, C₅+, eau, DEE, E2BE), celles-ci étant éliminées avec les différents produits issus du procédé (mélange C₄/éthanol en tête du débutaniseur, ETBE en fond du débutaniseur et mélange ABT/éthanol en fond de la colonne C2). Le procédé de la présente invention ne nécessite de ce fait pas de purge.

Une variante du procédé de l'invention consiste à remplacer le débutaniseur C1 par une colonne de distillation catalytique ou réactive C'1, ce qui permet d'augmenter les taux de conversion de l'isobutène et/ou de réduire l'excès d'éthanol employé. Les effluents de la colonne C'1 auront des compositions analogues à celles de ceux du débutaniseur C1 avec simplement des teneurs en éthanol généralement réduites.

Le procédé de purification d'ETBE à partir d'un mélange ETBE/éthanol/hydrocarbures, tel qu'il a été décrit ci-dessus, peut aisément être intégré dans un procédé de synthèse d'ETBE à partir d'éthanol et d'isobutène contenu dans une coupe C₄ pouvant par exemple provenir d'une unité de vapocraquage, de craquage catalytique ou de déshydrogénation des butanes.

Un tel procédé de synthèse d'ETBE est décrit ci-dessous en liaison avec la figure 2. Sur cette figure 2 on n'a pas représenté les divers équipements (pompes, échangeurs de chaleur, vannes de détente, ballons, ...) nécessaires au bon fonctionnement des différentes étapes du procédé.

La coupe C₄ contenant l'isobutène ainsi que des quantités mineures de C₃ et de C₅ est amenée en phase liquide par la ligne 20. L'éthanol d'appoint nécessaire à la réaction est amené en phase liquide par la ligne 21. L'azéotrope eau/éthanol produit par la section LD de lavage à l'eau du raffinat C₄ obtenu en tête du débutaniseur C1 (ou C'1) est recyclé en phase liquide par la ligne 24. Le mélange éthanol/ABT produit en fond de la zone de distillation C2 du procédé de l'invention est recyclé en phase liquide par la ligne 8. Ces divers fluides sont mélangés puis admis dans la section réactionnelle R par la ligne 22.

L'ABT présent dans l'effluent de la section réactionnelle provient de la réaction d'addition d'une molécule d'eau sur une molécule d'isobutène. L'eau est introduite dans le réacteur en raison du recyclage d'un azéotrope eau/éthanol à environ 5 % en poids d'eau, provenant de la section LD de lavage à l'eau du raffinat C₄ du débutaniseur C1 (ou C'1), mais également en raison de sa présence à l'état de traces dans l'éthanol d'appoint et dans la coupe C4 alimentant la section réactionnelle. Enfin, il y a dans la section réactionnelle elle-même formation d'eau par la réaction d'éthérification de l'éthanol conduisant à la formation d'une molécule d'eau et d'une molécule de DEE à partir de deux molécules d'éthanol.

La réaction d'addition conduisant à la formation d'ABT étant équilibrée thermodynamiquement le recyclage vers la section réactionnelle de l'ABT avec l'éthanol produit en fond de la zone de distillation C2 permet donc de limiter la conversion d'isobutène en ABT et, par là-même, d'accroître d'autant la conversion d'isobutène en ETBE, ce qui est avantageux du point de vue du procédé.

La section réactionnelle R produit par la ligne 1 un mélange constitué de l'ETBE produit, des hydrocarbures C₄ non réactifs et non réagis, de l'excès d'éthanol n'ayant pas réagi ainsi que des diverses impuretés déjà mentionnées (ABT, eau, C₃, C₅+, DEE, E2BE). Ce mélange alimente le débutaniseur C1 (ou C'1).

Du débutaniseur C1 (ou C'1) on recueille en fond par ligne 2 l'ETBE purifié, avec les autres éthers à l'état de traces (DEE, E2BE) et en tête les hydrocarbures, l'eau et une proportion variable, généralement de 1 à 5 % en poids d'éthanol. Ce raffinat une fois liquéfié est admis par la ligne 4 dans la section de lavage à l'eau LD.

Cette section de lavage à l'eau LD est en fait constituée d'une étape de lavage à l'eau proprement dit produisant par la ligne 23 les hydrocarbures essentiellement exempts d'éthanol, ainsi qu'un mélange eau/éthanol, et d'une étape de distillation dudit mélange produisant en fond de l'eau recyclée vers le lavage à l'eau et en tête un mélange azéotropique eau/éthanol contenant environ 5 % en poids d'eau, qui est recyclé par la ligne 24 vers la section réactionnelle R.

Au moins une phase vapeur, liquide ou mixte est soutirée latéralement du débutaniseur C1 (ou C'1) et amenée par la ligne 6 dans la zone de distillation C2.

La zone de distillation C2 produit en tête un mélange d'éthanol, d'ETBE et des autres produits dans des proportions variables, qui est recyclé par la ligne 11 vers le débutaniseur C1 (ou C'1), et en fond un mélange d'éthanol et d'ABT qui est recyclé par la ligne 8 vers la section réactionnelle R.

Dans le cas où le débutaniseur C1 est remplacé par une colonne de distillation catalytique ou réactive C'1, il peut être avantageux de recycler au moins partiellement le mélange éthanol/ABT obtenu en fond de la zone distillation C2 vers ladite distillation catalytique ou réactive C'1, par les lignes 25 et 27. Il peut de même être avantageux de recycler au moins partiellement le mélange azéotropique eau/éthanol produit par la section de lavage à l'eau LD, par les lignes 26 et 27 vers ladite colonne de distillation catalytique ou réactive C'1. Ladite ligne 27 peut alors avantageusement être disposée de manière à ce que l'éthanol ainsi recyclé soit admis au niveau d'un (ou des) plateau(x) catalytique(s) de C'1.

Dans certains cas, il peut être avantageux de recycler au moins partiellement le mélange azéotropique eau/éthanol produit par la section de lavage à l'eau LD par les lignes 29 et 30 et/ou le mélange éthanol/ABT produit en fond de la zone de distillation C2 par les lignes 28 et 30, vers le débutaniseur C1 (ou C'1) et ce de manière à augmenter la valeur de la concentration en éthanol au plateau dudit débutaniseur C1 (ou C'1) où s'effectue le soutirage latéral alimentant C2 afin de faciliter la séparation effectuée dans la zone de distillation C2. Ladite ligne 30 peut alors avantageusement être disposée de manière à ce que l'éthanol ainsi recyclé soit admis au voisinage du (ou des) plateau(x) au(x)quel(s) est effectué le soutirage latéral.

Enfin le procédé de la présente invention, tel qu'il a été décrit pour la purification de l'ETBE, peut également être intégré dans des procédés de synthèse d'autres éthers à partir de monoalcools aliphatiques ayant au moins 2 atomes de carbone et d'isooléfines ayant au moins 4 atomes de carbone, tels que, par exemple, des procédés de synthèse d'IPTBE ou d'ETAE.

L'exemple suivant illustre l'invention.

### Exemple

On a réalisé la séparation d'un mélange C₄/éthanol/ETBE représentatif d'un effluent de section réactionnelle d'un procédé d'éthérification de manière à obtenir la coupe C₄ en tête du débutaniseur (C1) à moins de 1 ppm en poids d'ETBE, l'ETBE en fond du débutaniseur à 0,1 % en poids d'éthanol résiduel et le mélange d'alcools (éthanol/ABT) en fond de la colonne de distillation C2 à 0,1 % en poids d'ETBE.

La composition et le débit du mélange à séparer (noté ALIMENTATION) sont donnés dans le tableau ci-dessous.

Le débutaniseur C1 est constitué d'une colonne en acier inoxydable d'un diamètre intérieur de 100 mm, comportant 70 plateaux perforés à déversoir, espacés de 5 cm.

La colonne de distillation C2 est constituée d'une colonne en verre d'un diamètre intérieur de 50 mm, comportant 30 plateaux perforés à déversoir, espacés de 5 cm .

Les deux colonnes sont isolées thermiquement de manière à éviter toutes les déperditions de calories et munies, chacunes, d'un bouilleur chauffé électriquement (respectivement R1 et R2), d'un condenseur à eau froide (respectivement E1 et E4) et d'un ballon de reflux (respectivement B1 et B2). En outre la colonne C2 est partiellement rebouillie par la chaleur de condensation de la vapeur soutirée latéralement sur la colonne C1, dans un échangeur E2.

Les plateaux des colonnes de distillation sont numérotés par ordre croissant à partir de 1 du haut vers le bas de chaque colonne.

Le débutaniseur C1 est opéré sous une pression, mesurée au ballon de reflux B1, de 8,0 bar. La température de la colonne C1 s'étage entre 161,0°C en fond et 67,5°C en tête. Le distillat obtenu en tête du débutaniseur C1 est condensé, puis sous-refroidi, ainsi la température dans le ballon de reflux B1 est-elle maintenue à 50°C.

Le mélange à séparer, ALIMENTATION, est introduit en phase liquide dans le débutaniseur (C1) au plateau numéro 40 à une température de 76,4°C.

Du ballon de reflux B1, on soutire en phase liquide le mélange (noté HC) d'hydrocarbures distillés. Le débit et la composition de ce mélange HC sont donnés dans le tableau ci-dessous. La fraction liquide résiduelle obtenue au niveau du ballon B1, de même composition que le mélange HC, est envoyée en reflux en tête du débutaniseur C1 sous un débit de 3300g.h⁻¹ ce qui représente un taux de reflux massique par rapport à l'ALIMENTATION de 0,55.

En fond du débutaniseur on récupère en phase liquide l'ETBE avec les autres éthers purifiés (cette phase étant notée ETHERS). Le débit et la composition du produit ETHERS ainsi obtenu sont donnés dans le tableau ci-dessous.

Au plateau numéro 58 du débutaniseur C1, on soutire une phase vapeur (notée SOUT), à une température de 129,8°C. Le débit et la composition de cette phase vapeur sont donnés dans le tableau ci-dessous.

Cette phase vapeur est condensée dans l'échangeur E2 puis sous-refroidie jusqu'à une température de 58,8°C dans un échangeur à eau froide E3. Elle est ensuite détendue dans une vanne de détente D1, jusqu'à la pression régnant dans la colonne C2 et admise dans cette colonne au plateau numéro 8.

La colonne de distillation C2 est opérée sous une pression de 1,1 bar, mesurée au ballon de reflux B2. La température de la colonne C2 s'étage entre 93,9°C en fond et 63,7°C en tête. La température au ballon de reflux B2 est de 38,3°C.

Du ballon de reflux B2, on soutire en phase liquide un mélange (noté RECYC), que l'on recycle, après l'avoir porté à la pression régnant dans le débutaniseur C1 et à une température de 117°C, au plateau numéro 58 du débutaniseur C1. Le débit et la composition de ce mélange RECYC sont donnés dans le tableau ci-dessous.

La fraction liquide résiduelle obtenue au niveau du ballon B2, de même composition que le mélange RECYC, est envoyée en reflux en tête de la colonne de distillation C2 sous un débit de 2087 g.h⁻¹, ce qui représente un taux de reflux massique par rapport à l'alimentation (SOUT) de la colonne de distillation C2 de 0,39.

En fond de la colonne de distillation C2, on récupère en phase liquide un mélange d'alcools (noté ALCOOLS, essentiellement constitué d'éthanol et d'ABT) dont le débit et la composition sont donnés dans le tableau ci-dessous.

**Tableau**

| **bilan matière du procédé de l'invention** | | | | | | |
|---|---|---|---|---|---|---|
| CORPS (% poids) | ALIMENTATION | ETHERS | HC | SOUT | RECYC | ALCOOLS |
| C3 | 1,24 | < 1 ppm | 1,82 | < 1 ppm | < 1 ppm | < 1 ppm |
| C4 | 64,87 | 0,02 | 94,85 | 5,61 | 5,77 | < 1 ppm |
| C5+ | 1,00 | 1,04 | 1,01 | 24,22 | 24,93 | < 1 ppm |
| Eau | 0,14 | < 1 ppm | 0,20 | < 1 ppm | < 1 ppm | < 1 ppm |
| Ethanol | 3,72 | 0,10 | 2,11 | 18,08 | 16,02 | 88,46 |
| ABT | 0,29 | 0,01 | 28 ppm | 0,54 | 0,22 | 11,44 |
| ETBE | 28,63 | 98,50 | < 1 ppm | 49,24 | 50,68 | 0,10 |
| DEE | 0,05 | 0,13 | 77 ppm | 2,31 | 2,38 | < 1 ppm |
| E2BE | 0,06 | 0,20 | < 1 ppm | < 1 ppm | < 1 ppm | < 1 ppm |
| Débit (g.h⁻¹) | 6000 | 1744 | 4104 | 5352 | 5200 | 152 |

## Revendications

1. Procédé de purification d'un éther formé entre un monoalcool aliphatique ayant au moins 2 atomes de carbone et une isooléfine ayant au moins 4 atomes de carbone, à partir d'un mélange contenant ledit éther, ledit monoalcool et en outre au moins des hydrocarbures dont le nombre d'atomes de carbone est égal à celui de ladite isooléfine et dans lequel on introduit ledit mélange dans une première zone de distillation, d'où on recueille en tête la quasi-totalité des hydrocarbures, ledit procédé étant caractérisé en ce qu'il comprend les étapes suivantes :
(a) on recueille, en fond, l'éther purifié ;
(b) on soutire latéralement de ladite première zone de distillation au moins une phase que l'on envoie dans une seconde zone de distillation, opérant à une pression inférieure à celle de ladite première zone de distillation, d'où on recueille, en fond, du monoalcool purifié et, comme effluent de tête, un mélange de monoalcool, d'éther et d'hydrocarbures, que l'on recycle vers ladite première zone de distillation.

2. Procédé selon la revendication 1 dans lequel on traite un mélange d'éthyl tertiobutyl éther (ETBE) et d'éthanol renfermant en outre au moins des hydrocarbures en C₄, ledit procédé étant caractérisé en ce qu'il comprend les étapes suivantes :
(a) on introduit le mélange à séparer dans une première zone de distillation dite zone de débutanisation, d'où on recueille, en tête, la quasi-totalité des hydrocarbures en C₄ et, en fond, l'ETBE purifié ;
(b) on soutire latéralement de ladite première zone de distillation au moins une phase que l'on envoie dans une seconde zone de distillation, opérant à une pression inférieure à celle de ladite première zone de distillation, d'où on recueille, en fond, de l'éthanol purifié et, comme effluent de tête, un mélange d'éthanol et d'ETBE que l'on recycle vers ladite première zone de distillation.

3. Procédé selon la revendication 2, caractérisé en ce que le soutirage latéral est effectué sur ladite première zone de distillation au niveau d'au moins un plateau dans lequel la concentration en alcool est sensiblement maximale.

4. Procédé selon l'une des revendications 2 et 3, caractérisé en ce que l'effluent de tête de ladite seconde zone de distillation, condensé, est au moins en partie recyclé, éventuellement après réchauffage, vers la première zone de distillation à un plateau de celle-ci situé au dessous de (ou confondu avec) le plateau de soutirage.

5. Procédé selon l'une des revendications 2 à 4, caractérisé en ce que la partie de l'effluent de tête de la seconde zone de distillation, condensé, recyclé vers la première zone de distillation est réchauffé et partiellement vaporisé, une fraction liquide et une fraction vapeur sont séparées, on admet ladite fraction liquide dans la première zone de distillation à un plateau de celle-ci au-dessous de (ou confondu avec) le plateau de soutirage; et on admet ladite fraction vapeur dans ladite première zone de distillation à un plateau situé au-dessus de celui auquel est admise ladite fraction liquide.

6. Procédé selon l'une des revendications 2 à 5, caractérisé en ce que dans ladite seconde zone de distillation, on opère à une pression inférieure d'au moins 1 bar, de préférence de 2 à 10 bar, à celle à laquelle on opère dans ladite première zone de distillation.

7. Procédé selon l'une des revendications 2 à 6, caractérisé en ce que, dans l'étape (a), la première zone de distillation opère sous une pression supérieure à la pression atmosphérique, de préférence comprise entre 5 et 15 bar, à une température de fond de 70 à 200 °C, à une température de tête de 30 à 100 °C, le mélange à séparer est introduit dans ladite zone de distillation au plateau ayant la composition la plus voisine possible de celle dudit mélange ; et en ce que, dans l'étape (b), la phase à envoyer vers la seconde zone de distillation est soutirée au niveau d'un plateau de ladite zone de distillation correspondant sensiblement à la concentration maximale en éthanol, elle est détendue à la pression de la seconde zone de distillation de 0.5 à 10 bar, la température de fond de ladite seconde zone de distillation étant de 70 à 170 °C et sa température de tête étant de 10 à 130 °C.

8. Procédé selon la revendication 7, caractérisé en ce que dans l'étape (b) ladite phase soutirée est une phase vapeur, soutirée au niveau d'un plateau de ladite première zone de distillation où la concentration en éthanol dans la phase vapeur est sensiblement maximale, et elle est au moins partiellement condensée avant d'être admise dans ladite seconde zone de distillation.

9. Procédé selon la revendication 8, caractérisé en ce que au moins une partie de la chaleur de condensation de la phase vapeur soutirée sur la première zone de distillation permet de rebouillir au moins en partie la seconde zone de distillation.

10. Procédé selon l'une des revendications 2 à 9, caractérisé en ce que le mélange à séparer provient d'une zone de réaction d'éthérification entre l'isobutène et l'éthanol pour la synthèse de l'ETBE.

11. Procédé selon la revendication 10, caractérisé en ce que le mélange ETBE/éthanol à séparer contient de 0.1 à 20 % en poids d'éthanol et de 5 à 80 % en poids d'ETBE, le reste étant principalement constitué d'hydrocarbures en C₄, ainsi que de proportions allant jusqu'à 1 % en poids d'eau, jusqu'à 1 % en poids d'alcool butylique tertiaire (ABT), jusqu'à 1 % en poids de diéthyléther (DEE), jusqu'à 1 % en poids d'éthyl 2-butyl éther (E2BE), jusqu'à 5 % en poids d'hydrocarbures ayant au moins 5 atomes de carbone (C₅+) et jusqu'à 5 % en poids d'hydrocarbures en C₃.

12. Procédé selon la revendication 11, caractérisé en ce que, dans l'étape (a), l'effluent de tête de la première zone de distillation comprend la majeure partie des hydrocarbures en C₃ et en C₄, la majeure partie de l'eau, une fraction de l'éthanol et des hydrocarbures C₅+, du mélange à séparer et est substantiellement exempt d'ETBE, d'E2BE, d'ABT et de DEE ; l'effluent de fond de ladite première zone de distillation comprend, outre l'ETBE purifié, la majeure partie de l'E2BE, une partie du DEE, une fraction des hydrocarbures C₅+ et moins de 1 % en poids d'éthanol et d'ABT ; et l'effluent de fond de la seconde zone de distillation consiste essentiellement en éthanol et ABT.

13. Procédé selon l'une des revendications 2 à 11, caractérisé en ce que l'ETBE purifié recueilli en fond de la première zone de distillation contient moins de 1 % en poids d'alcool(s).

14. Procédé selon les revendications 2 à 13, caractérisé en ce que l'ETBE purifié recueilli en fond de la première zone de distillation contient moins de 0,1 % en poids d'alcool(s).

15. Procédé de synthèse d'éther à partir d'un monoalcool aliphatique ayant au moins 2 atomes de carbone et d'une isooléfine ayant au moins 4 atomes de carbone, contenue dans une coupe d'hydrocarbures comprenant une proportion prépondérante d'hydrocarbures ayant le même nombre d'atomes de carbone que l'isooléfine, caractérisé en ce que l'effluent de la section réactionnelle est traité selon le procédé de la revendication 1, et en ce que l'alcool obtenu en fond de la seconde zone de distillation est renvoyé vers ladite section réactionnelle.

16. Procédé de synthèse d'ETBE à partir d'éthanol et de l'isobutène contenu dans une coupe C₄ caractérisé en ce qu'il comprend les étapes suivantes :
(1) on mélange un flux de coupe C₄, contenant des proportions mineures d'hydrocarbures en C₃ et C₅+ un appoint d'éthanol, de l'azéotrope eau-éthanol provenant de la section de lavage (LD) du raffinat obtenu en tête de la première zone de distillation (C1) et l'effluent de fond de la seconde zone de distillation (C2), et on admet le mélange ainsi formé dans une section réactionnelle (R), qui opère dans des conditions d'éthérification de l'isobutène et de l'éthanol ;
(2) on alimente la première zone de distillation (C1) au moyen de l'effluent de la zone réactionnelle (R) qui comprend l'ETBE produit, les hydrocarbures en C₄ non réactifs ou non réagis, l'excès d'éthanol et diverses impuretés : ABT, eau, hydrocarbures en C₃ et C₅+, DEE et E2BE;
(3) on traite ledit effluent de la zone réactionnelle (R) en tant que mélange à séparer par le procédé selon l'une des revendications 2 à 14, l'effluent de fond de ladite seconde zone de distillation (C2) étant recyclé vers la section réactionnelle (R);
(4) on envoie l'effluent vapeur de tête de la première zone de distillation (C1), après condensation, vers une section de lavage à l'eau (LD), qui produit un effluent constitué d'hydrocarbures essentiellement exempts d'éthanol et un mélange eau-éthanol dont on sépare, par distillation, an fond, de l'eau, que l'on recycle vers le lavage et, en tête, un mélange azéotropique eau/éthanol à environ 5 % en poids d'eau, que l'on recycle vers la section réactionnelle (R).

17. Procédé selon la revendication 16, caractérisé en ce que la zone de distillation (C1) est remplacée par une zone de distillation catalytique ou réactive (C'1).

18. Procédé selon la revendication 17, caractérisé en ce que au moins une partie du résidu de fond de la seconde zone de distillation (C2) de l'étape (3) est recyclée vers ladite zone de distillation catalytique ou réactive (C'1), à un niveau correspondant à un plateau catalytique.

19. Procédé selon l'une des revendications 17 et 18, caractérisé en ce que au moins une partie du mélange azéotropique eau/éthanol, produit par la zone de lavage-distillation (LD) de l'étape (4) est recyclée vers ladite zone de distillation catalytique ou réactive (C'1), à un niveau correspondant à un plateau catalytique.

20. Procédé selon l'une des revendications 16 à 19, caractérisé en ce que au moins une partie du résidu de fond de la seconde zone de distillation (C2) de l'étape (3) est recyclée vers ladite zone de distillation (C1) ou vers ladite zone de distillation catalytique ou réactive (C'1), à un niveau voisin du soutirage sur ladite zone.

21. Procédé selon l'une des revendications 16 à 20, caractérisé en ce que au moins une partie du mélange azéotropique eau/éthanol, produit par la zone de lavage-distillation (LD) de l'étape (4) est recyclée vers ladite zone de distillation (C1) ou vers ladite zone de distillation catalytique ou réactive (C'1), à un niveau voisin du soutirage sur ladite zone.

## Claims

1. A process for the purification of an ether formed from an aliphatic monoalcohol containing at least two carbon atoms and an isoolefin containing at least four carbon atoms, from a mixture containing said ether, said monoalcohol and hydrocarbons containing the same number of carbon atoms as that in said isoolefin, and in which said mixture is introduced, into a first distillation zone, from which almost all of the hydrocarbons are recovered overhead, said process being characterised in that it comprises the following steps :
(a) the purified ether is recovered from the bottom ;
(b) at least one phase is extracted as a side stream from said first distillation zone and is sent to a second distillation zone, operating at a lower pressure than that of said first distillation zone, from which purified monoalcohol is recovered from the bottom and a mixture of monoalcohol, ether and hydrocarbons is recovered as an overhead effluent and recycled to said first distillation zone.

2. A process according to claim 1, in which a mixture of ethyl tertio-butyl ether (ETBE) and ethanol which also contains at least C₄ hydrocarbons is treated, said process being characterised in that it comprises the following steps:
(a) the mixture to be separated is introduced into a first distillation zone, the debutanising zone, from which almost all of the C₄ hydrocarbons are recovered overhead and the purified ETBE is recovered from the bottom;
(b) at least one phase is extracted as a side stream from said first distillation zone and sent to a second distillation zone, operating at a lower pressure than that of said first distillation zone, from which purified ethanol is recovered from the bottom and a mixture of ethanol and ETBE and hydrocarbons is recovered as an overhead effluent and recycled to said first distillation zone.

3. A process according to claim 2, characterised in that the side stream is extracted from said first distillation zone from at least one tray in which the alcohol concentration is substantially at a maximum.

4. A process according to any one of claims 2 and 3, characterised in that the condensed overhead effluent from said second distillation zone is recycled at least in part, if necessary after reheating, to the first distillation zone to a tray thereof which is located below (or at the same level as) the tray at which extraction is effected.

5. A process according to any one of claims 2 to 4, characterised in that the portion of the condensed overhead effluent from the second distillation zone which is recycled to the first distillation zone is reheated and partially vaporised, a liquid fraction and a vapour fraction are separated, said liquid fraction is admitted to the first distillation zone at a tray thereof which is below (or at the same level as) the tray at which extraction is effected; and said vapour fraction is admitted to said first distillation zone at a tray which is located above that at which said liquid fraction is admitted.

6. A process according to any one of claims 2 to 5, characterised in that the second distillation zone is operated at a pressure which is at least 1 bar, preferably 2 to 10 bars, lower than that at which said first distillation zone is operated.

7. A process according to any one of claims 2 to 6, characterised in that, in step (a), the first distillation zone is operated at a pressure which is above atmospheric pressure, preferably between 5 and 15 bar, at a bottom temperature of 70°C to 200°C, at a top temperature of 30°C to 100°C, the mixture to be separated is introduced into said distillation zone to the tray with the closest composition to that of said mixture; and in that, in step (b), the phase to be sent to the second distillation zone is extracted from a tray in said distillation zone which substantially corresponds to the maximum ethanol concentration, and it is depressurised to the pressure of the second distillation zone of 0.5 to 10 bar, the bottom temperature of said second distillation zone being 70°C to 170°C and the top temperature being 10°C to 130°C.

8. A process according to claim 7, characterised in that in step (b), said extracted phase is a vapour phase extracted from the tray in said first distillation zone where the ethanol concentration in the vapour phase is substantially at a maximum, and it is at least partially condensed before admission into said second distillation zone.

9. A process according to claim 8, characterised in that at least a portion of the heat of condensation of the vapour phase extracted from the first distillation zone is used for reboiling, at least in part, the second distillation zone.

10. A process according to any one of claims 2 to 9, characterised in that the mixture to be separated is from an etherification zone reacting isobutene and ethanol to synthesize ETBE.

11. A process according to claim 10, characterised in that the ETBE/ethanol mixture to be separated contains 0.1% to 20% by weight of ethanol and 5% to 80% by weight of ETBE, the remainder being principally constituted by C₄ hydrocarbons, and proportions of up to 1% by weight of water, up to 1% by weight of tertiary butyl alcohol (TBA), up to 1% by weight of diethyl ether (DEE), up to 1% by weight of ethyl 2-butyl ether (E2BE), up to 5% by weight of hydrocarbons containing at least 5 carbon atoms (C₅+) and up to 5% by weight of C₃ hydrocarbons.

12. A process according to claim 11, characterised in that, in step (a), the overhead effluent from the first distillation zone comprises the major portion of C₃ and C₄ hydrocarbons, the major portion of the water, a fraction of the ethanol and C₅+ hydrocarbons, of the mixture to be separated, and is substantially free of ETBE, E2BE, TBA and DEE; the bottom effluent from said first distillation zone comprises, apart from purified ETBE, the major portion of the E2BE, a portion of the DEE, a fraction of the C₅+ hydrocarbons and less than 1% by weight of the ethanol and the TBA; and the bottom effluent from the second distillation zone essentially consists of ethanol and TBA.

13. A process according to any one of claims 2 to 11, characterised in that the purified ETBE recovered from the bottom of the first distillation zone contains less than 0.1% by weight of alcohol(s).

14. A process according to claims 2 to 13, characterised in that the purified ETBE recovered from the bottom of the first distillation zone contains less than 0.1% by weight of alcohol(s).

15. A process for the synthesis of an ether from an aliphatic monoalcohol containing at least two carbon atoms and an isoolefin containing at least four carbon atoms, contained in a hydrocarbon cut comprising a major proportion of hydrocarbons containing the same number of carbon atoms as the isoolefin, characterised in that the effluent from the reaction section is treated using the process of claim 1, and in that the alcohol obtained from the bottom of the second distillation zone is returned to said reaction section.

16. A process for the synthesis of ETBE from ethanol and isobutene contained in a C₄ cut, characterised in that it comprises the following steps:
(1) a C₄ cut stream containing minor proportions of C₃ and C₅+ hydrocarbons, additional ethanol, a water/ethanol azeotrope from a washing section (LD) for the raffinate obtained overhead from a first distillation zone (C1) and the bottom effluent from a second distillation zone (C2) are mixed and admitting the mixture formed into a reaction section (R) which operates under conditions suitable for the etherficiation of isobutene and ethanol ;
(2) the first distillation zone (C1) is supplied with the effluent from the reaction zone (R), which comprises the ETBE produced, non reactive or unreacted C₄ Hydrocarbons, excess ethanol and various impurities : TBA, water, C₃ and C₅+ hydrocarbons, DEE and E2BE ;
(3) said effluent from the reaction zone (R) is treated as the mixture to be separated by the process according to any one of claims 2 to 14, the bottom effluent from said second distillation zone (C2) being recycled to reaction section (R) ;
(4) the overhead vapour phase effluent from the first distillation zone (C1) is sent, after condensing, to a water washing section (LD), to produce an effluent constituted by hydrocarbons which are essentially free of ethanol, and a water/ethanol mixture which is separated by distillation into a water bottoms product which is recycled to the washing section and an azeotropic water/ethanol mixture containing about 5 % by weight of water as an overhead product, which is recycled to the reaction section (R).

17. A process according to claim 16, characterised in that the distillation zone (C1) is replaced by a catalytic or reactive distillation zone (C'1).

18. A process according to claim 17, characterised in that at least a portion of the bottom residue from the second distillation zone (C2) of step (3) is recycled to said catalytic or reactive distillation zone (C'1), to a tray corresponding to a catalytic tray.

19. A process according to any one of claims 17 and 18, characterised in that at least a portion of the azeotropic water/ethanol mixture produced by the washing-distillation zone (LD) of step (4) is recycled to said catalytic or reactive distillation zone (C'1), to a level corresponding to a catalytic tray.

20. A process according to any one of claims 16 to 19, characterised in that at least a portion of the residue from the bottom of the second distillation zone (C2) in step (3) is recycled to said distillation zone (C1) or to said catalytic or reactive distillation zone (C'1), to a level close to that at which the extraction is made from said zone.

21. A process according to any one of claims 16 to 20, characterised in that at least a portion of the azeotropic water/ethanol mixture produced by the washing-distillation zone (LD) of step (4) is recycled to said distillation zone (C1) or to said catalytic or reactive distillation zone (C'1) to a level close to that at which the extraction is made from said zone.

## Patentansprüche

1. Verfahren zum Reinigen eines zwischen einem aliphatischen Monoalkohol mit wenigstens 2 Kohlenstoffatomen und einem Isoolefin mit wenigstens 4 Kohlenstoffatomen gebildeten Ethers aus einem Gemisch, das den Ether, den Monoalkohol und ferner wenigstens Kohlenwasserstoffe, deren Kohlenstoffatomzahl gleich der des Isoolefins ist, enthält, und bei dem das Gemisch in eine erste Destillationszone eingeführt wird, von wo am Kopf die Quasi-Gesamtheit der Kohlenwasserstoffe aufgefangen wird, dadurch gekennzeichnet, daß es folgende Schritte umfaßt:
(a) Auffangen des gereinigten Ethers am Boden;
(b) seitliches Abzapfen, von der ersten Destillationszone, wenigstens einer Phase, die in eine zweite Destillationszone geschickt wird, die bei einem niedrigeren Druck als dem der ersten Destillationszone arbeitet, von wo am Boden gereinigter Monoalkohol und als Kopfeffluent ein Gemisch von Monoalkohol, Ether und Kohlenwasserstoffen aufgefangen wird, das zu der ersten Destillationszone zurückgeführt wird.

2. Verfahren nach Anspruch 1, bei dem ein Gemisch von Ethyl-Tert-Butylether (ETBE) und Ethanol behandelt wird, das ferner wenigstens C₄-Kohlenwasserstoffe enthält, wobei das Verfahren durch folgende Schritte gekennzeichnet ist:
(a) Einführen des zu trennenden Gemischs in eine als Debutanisierungszone bezeichnete erste Destillationszone, von wo am Kopf die Quasi-Gesamtheit der C₄-Kohlenwasserstoffe und am Boden das gereinigte ETBE aufgefangen wird;
(b) seitliches Abzapfen, von der ersten Destillationszone, wenigstens einer Phase, die in eine zweite Destillationszone geschickt wird, die bei einem niedrigeren Druck als dem der ersten Destillationszone arbeitet, von wo am Boden gereinigtes Ethanol und als Kopfeffluent ein Gemisch von Ethanol und ETBE aufgefangen wird, das zur ersten Destillationszone zurückgeführt wird.

3. Verfahren nach Anspruch 2, dadurch gekennzeichnet, daß die seitliche Abzapfung an der ersten Destillationszone in Höhe wenigstens eines Bodens durchgeführt wird, auf dem die Alkoholkonzentration im wesentlichen maximal ist.

4. Verfahren nach einem der Ansprüche 2 und 3, dadurch gekennzeichnet, daß der kondensierte Kopfeffluent der zweiten Destillationszone wenigstens teilweise, eventuell nach Nacherhitzung, zur ersten Destillationszone auf einen Boden von dieser zurückgeführt wird, der unterhalb des Abzapfungsbodens liegt (oder mit diesem zusammenfällt).

5. Verfahren nach einem der Ansprüche 2 bis 4, dadurch gekennzeichnet, daß der kondensierte und zur ersten Destillationszone zurückgeführte Anteil des Kopfeffluenten der zweiten Destillationszone nacherhitzt und teilweise verdampft wird, eine flüssige Fraktion und eine Dampffraktion getrennt werden, die flüssige Fraktion in die erste Destillationszone auf einem Boden von dieser unterhalb des Abzapfungsbodens (oder mit diesem zusammenfallend) eingelassen wird; und daß die Dampffraktion in die erste Destillationszone auf einem Boden eingelassen wird, der über demjenigen liegt, an dem die flüssige Fraktion eingelassen wird.

6. Verfahren nach einem der Ansprüche 2 bis 5, dadurch gekennzeichnet, daß in der zweiten Destillationszone bei einem Druck gearbeitet wird, der um wenigstens 1 bar, vorzugsweise 2 bis 10 bar niedriger ist als der, bei dem in der ersten Destillationszone gearbeitet wird.

7. Verfahren nach einem der Ansprüche 2 bis 6, dadurch gekennzeichnet, daß in Schritt (a) die erste Destillationszone unter einem Druck über Atmosphärendruck, vorzugsweise zwischen 5 und 15 bar, bei einer Fußtemperatur von 70 bis 200°C, bei einer Kopftemperatur von 30 bis 100°C arbeitet, das zu trennende Gemisch in die Destillationszone auf dem Boden eingeführt wird, der die dem Gemisch engstmöglich benachbarte Zusammensetzung hat; und daß in Schritt (b) die in die zweite Destillationszone zu schickende Phase in Höhe eines Bodens der Destillationszone abgezapft wird, der im wesentlichen der maximalen Ethanolkonzentration entspricht, sie auf den Druck der zweiten Destillationszone von 0,5 bis 10 bar entspannt wird, wobei die Fußtemperatur der zweiten Destillationszone bei 70 bis 170°C und ihre Kopftemperatur bei 10 bis 130°C liegt.

8. Verfahren nach Anspruch 7, dadurch gekennzeichnet, daß in Schritt (b) die abgezapfte Phase eine Dampfphase ist, die in Höhe eines Bodens der ersten Destillationszone abgezapft wird, wo die Ethanolkonzentration in der Dampfphase im wesentlichen maximal ist, und daß sie wenigstens teilweise kondensiert wird, bevor sie in die zweite Destillationszone eingelassen wird.

9. Verfahren nach Anspruch 8, dadurch gekennzeichnet, daß wenigstens ein Teil der Kondensationswärme der an der ersten Destillationszone abgezapften Dampfphase es gestattet, die zweite Destillationszone wenigstens teilweise abzukochen.

10. Verfahren nach einem der Ansprüche 2 bis 9, dadurch gekennzeichnet, daß das zu trennende Gemisch aus einer Etherisierungsreaktionszone zwischen Isobuten und Ethanol zur ETBE-Synthese kommt.

11. Verfahren nach Anspruch 10, dadurch gekennzeichnet, daß das zu trennende ETBE/Ethanol-Gemisch 0,1 bis 20 Gew.% Ethanol und 5 bis 80 Gew.% ETBE enthält, wobei der Rest hauptsächlich gebildet ist durch C₄-Kohlenwasserstoffe sowie Anteile von bis zu 1 Gew.% Wasser, bis zu 1 Gew.% Tert-Butanol (ABT), bis zu 1 Gew.% Diethylether (DEE), bis zu 1 Gew.% Ethyl-2-Butylether (E2BE), bis zu 5 Gew.% Kohlenwasserstoffe mit mindestens 5 Kohlenstoffatomen (C₅₊) und bis zu 5 Gew.% C₃-Kohlenwasserstoffen.

12. Verfahren nach Anspruch 11, dadurch gekennzeichnet, daß im Schritt (a) der Kopfeffluent des ersten Destillationsabschnitts den größten Teil der C₃- und C₄-Kohlenwasserstoffe, den größten Teil des Wassers, einen Bruchteil des Ethanols und der C₅₊-Kohlenwasserstoffe des zu trennenden Gemischs enthalt und im wesentlichen frei ist von ETBE, E2BE, ABT und DEE; daß der Fußeffluent der ersten Destillationszone außer dem gereinigten ETBE den größten Teil des E2BE, einen Teil des DEE, einen Bruchteil der C₅₊-Kohlenwasserstoffe und weniger als 1 Gew.% Ethanol und ABT umfaßt; und der Fußeffluent der zweiten Destillationszone im wesentlichen aus Ethanol und ABT besteht.

13. Verfahren nach einem der Ansprüche 2 bis 11, dadurch gekennzeichnet, daß das am Fuß der ersten Destillationszone aufgefangene gereinigte ETBE weniger als 1 Gew.% Alkohol(e) enthält.

14. Verfahren nach den Ansprüchen 2 bis 13, dadurch gekennzeichnet, daß das am Fuß der ersten Destillationszone aufgefangene gereinigte ETBE weniger als 0,1 Gew.% Alkohol(e) enthält.

15. Verfahren zur Synthese von Ether aus einem aliphatischen Monoalkohol mit wenigstens 2 Kohlenstoffatomen und einem Isoolefin mit wenigstens 4 Kohlenstoffatomen, enthalten in einem Kohlenwasserstoffschnitt mit einem überwiegenden Anteil von Kohlenwasserstoffen mit derselben Kohlenstoffatomzahl wie das Isoolefin, dadurch gekennzeichnet, daß der Effluent des Reaktionsabschnitts nach dem Verfahren nach Anspruch 1 behandelt wird, und daß der am Fuß der zweiten Destillationszone erhaltene Alkohol in den Reaktionsabschnitt zurückgeschickt wird.

16. Verfahren zur Synthese von ETBE aus Ethanol und Isobuten, enthalten in einem C₄-Schnitt, dadurch gekennzeichnet, daß es folgende Schritte umfaßt:
(1) Mischen eines C₄-Schnitt-Flusses, der kleinere Anteile C₃- und C₅₊-Kohlenwasserstoffe enthält, eines Ethanoldurchsatzes, von Wasser-Ethanol-Azeotrop, kommend aus dem waschabschnitt (LD) des am Kopf der ersten Destillationszone (C1) erhaltenen Raffinats, und des Fußeffluenten der zweiten Destillationszone (C2), und Einlassen des so gebildeten Gemischs in einen Reaktionsabschnitt (R), der unter Bedingungen der Etherisierung des Isobutens und des Ethanols arbeitet;
(2) Versorgen der ersten Destillationszone (C1) mit Hilfe des Effluenten der Reaktionszone (R), der das erzeugte ETBE, die nicht-reaktiven oder nicht-umgesetzten C₄-Kohlenwasserstoffe, den Ethanolüberschuß und diverse Verunreinigungen: ABT, Wasser, C₃- und C₅₊-Kohlenwasserstoffe, DEE und E2BE, enthält;
(3) Behandeln des Effluenten der Reaktionszone (R) als zu trennendes Gemisch mit dem Verfahren nach einem der Ansprüche 2 bis 14, wobei der Fußeffluent der zweiten Destillationszone (C2) zum Reaktionsabschnitt (R) zurückgeführt wird;
(4) Leiten des dampfförmigen Kopfeffluenten der ersten Destillationszone (C1) nach Kondensation zu einem Wasser-Waschabschnitt (LD), der einen Effluenten, bestehend aus im wesentlichen ethanolfreien Kohlenwasserstoffen und ein Wasser-Ethanolgemisch erzeugt, von dem man durch Destillation am Fuß Wasser, das zum Waschen zurückgeführt wird, und am Kopf ein azeoptropes Wasser-Ethanolgemisch mit ca. 5 Gew.% Wasser erzeugt, das zum Reaktionsabschnitt (R) zurückgeführt wird.

17. Verfahren nach Anspruch 16, dadurch gekennzeichnet, daß die Destillationszone (C1) durch eine katalytische oder reaktive Destillationszone (C'1) ersetzt ist.

18. Verfahren nach Anspruch 17, dadurch gekennzeichnet, daß wenigstens ein Teil des Fußrückstands der zweiten Destillationszone (C2) des Schritts (3) zur katalytischen oder reaktiven Destillationszone (C'1) an einer einem katalytischen Boden entsprechenden Höhe zurückgeführt wird.

19. Verfahren nach einem der Ansprüche 17 und 18, dadurch gekennzeichnet, daß wenigstens ein Teil des durch die Wasch-Destillationszone (LD) des Schritts (4) erzeugten azeotropen Wasser-Ethanol-Gemischs zur katalytischen oder reaktiven Reaktionszone (C'1) an einer einem katalytischen Boden entsprechenden Höhe zurückgeführt wird.

20. Verfahren nach einem der Ansprüche 16 bis 19, dadurch gekennzeichnet, daß wenigstens ein Teil des Fuß-Rückstands der zweiten Destillationszone (C2) des Schritts (3) zur Destillationszone (C1) oder zur katalytischen oder reaktiven Destillationszone (C'1) in einer der Abzapfung von dieser Zone benachbarten Höhe zurückgeführt wird.

21. Verfahren nach einem der Ansprüche 16 bis 20, dadurch gekennzeichnet, daß wenigstens ein Teil des durch den Wasch-Destillationsabschnitt (LD) des Schritts (4) erzeugten azeoptropen Wasser-Ethanolgemischs zur Destillationszone (C1) oder zur katalytischen oder reaktiven Reaktionszone (C'1) in einer der Abzapfung von dieser Zone benachbarten Höhe zurückgeführt wird.
